# FASCICULE DE BREVET EUROPEEN

(11) **EP 2 675 729 B1**
(45) Date de publication et mention de la délivrance du brevet: **21.09.2016**
(21) Numéro de dépôt: 12709935.6
(22) Date de dépôt: 17.02.2012
(51) Int. Cl.: B65D 83/54, B65D 83/38, A61M 15/00

(54) **DISPOSITIF DE DISTRIBUTION DE PRODUIT FLUIDE**
VORRICHTUNG FÜR DIE AUSGABE EINES FLUIDS
DEVICE FOR DISPENSING A FLUID MATERIAL

(30) Priorité: 17.02.2011 FR 1151287
(43) Date de publication de la demande: 25.12.2013
(73) Titulaire: Aptar France SAS, 27110 Le Neubourg (FR)
(72) Inventeur: JACUK, Christophe, F-27100 Le Vaudreuil (FR); PAPET, Gérard, F-76300 Sotteville les Rouen (FR)
(74) Mandataire: CAPRI
(86) Numéro de dépôt international: PCT/FR2012/050353
(87) Numéro de publication internationale: WO 2012/110751

(56) Documents cités:
- EP-A2- 1 878 507
- FR-A1- 2 738 557
- FR-A1- 2 948 645
- US-A1- 2008 230 567

## Description

La présente invention concerne un dispositif de distribution de produit fluide.

Plus particulièrement, la présente invention concerne un dispositif de distribution de produit fluide pharmaceutique du type inhalateur à dose mesurée, communément appelé pMDI (pressurized Metered Dose Inhaler). Dans ce type de dispositif, le produit fluide pharmaceutique, qui contient un ou plusieurs produit(s) actif(s), est associé à un gaz propulseur et disposé dans un réservoir sous pression. Une valve doseuse est assemblée sur le réservoir et est actionnée pour distribuer une dose de produit fluide à chaque actionnement. Les inhalateurs de ce type comportent un corps externe dans lequel le réservoir peut coulisser, généralement axialement, pour actionner la valve et distribuer la dose de produit fluide à travers un orifice de distribution, généralement un embout buccal. Ce type de dispositif est bien connu dans l'état de la technique, comme par exemple dans le document WO 99/51205 A1 qui décrit un dispositif selon le préambule de la revendication 1. Il existe de nombreux types de valves doseuses utilisables avec ce type d'inhalateur. De manière générale, une valve doseuse comporte un corps de valve dans lequel coulisse une soupape. Le corps de valve contient une chambre de dosage et lorsque la soupape est enfoncée, la chambre de dosage se vide à travers ladite soupape sous l'effet du gaz propulseur. Lorsqu'ensuite la soupape revient vers sa position de repos, une nouvelle dose est chargée dans la chambre de dosage. De manière connue, ce type d'inhalateur pMDI comporte un ou plusieurs élément(s) d'étanchéité. Les éléments d'étanchéité assurent l'étanchéité à différents endroits, et de manière classique, il existe généralement un joint de col, qui est interposé entre la valve doseuse, le réservoir et la bague de fixation qui sert à fixer la valve sur le réservoir. Par ailleurs, la valve elle-même comporte un ou plusieurs joint(s) d'étanchéité qui coopère(nt) avec la soupape lorsque celle-ci est au repos et/ou lorsqu'elle se déplace vers sa position actionnée. Dans les valves les plus courantes, la valve comporte généralement deux joints appelés « joints internes » contre lesquels la soupape va coulisser de manière étanche lors de l'actionnement. Ces différents éléments d'étanchéité sont donc susceptibles d'être en contact avec le produit actif contenu dans le produit fluide à distribuer. Ils sont également en contact avec le gaz propulseur. Généralement, ces éléments d'étanchéité sont réalisés en matériau élastomère, du type EPDM, nitrile, chloroprène, etc. Tous ces matériaux sont plus ou moins performants selon les propriétés considérées, et présentent tous certains inconvénients. En particulier, ils sont susceptibles d'interagir avec le produit actif et/ou le gaz propulseur. Il est donc souhaitable de trouver des matériaux pour réaliser ces éléments d'étanchéité qui interagissent le moins possible avec ledit produit actif et/ou ledit gaz propulseur, tout en étant facile à fabriquer et à assembler, pour s'adapter aux chaînes de montage à haute cadence typiques pour ces dispositifs d'inhalateur.

Le document WO 98/07768 décrit un matériau appelé COC élastomère (Cyclo Oléfine Copolymère Elastomère), qui a été développé pour réaliser notamment des tuyaux, tubes ou poches souples dans le domaine médical. Ce matériau n'a toutefois jamais été utilisé dans des dispositifs du type inhalateur, en particulier en contact avec des gaz propulseur qui ont une action très agressive sur les matériaux constitutifs des éléments d'étanchéité. Or, il a été constaté de manière surprenante que ce matériau COC élastomère s'avère particulièrement bénéfique et adapté à être utilisé dans ces applications de valve doseuse fonctionnant avec un gaz propulseur, notamment du type HFA.

La présente invention a donc pour but de fournir un dispositif de distribution de produit fluide qui ne reproduit pas les inconvénients susmentionnés.

Plus particulièrement, la présente invention a pour but de fournir un dispositif de distribution de produit fluide qui améliore les propriétés des éléments d'étanchéité utilisées dans le dispositif, et qui limite les interactions néfastes entre lesdits éléments d'étanchéité et le produit fluide et/ou le gaz propulseur avec lesquels il est en contact.

La présente invention a aussi pour but de fournir un dispositif de distribution de produit fluide qui soit simple et peu coûteux à fabriquer et à assembler.

La présente invention a donc pour objet un dispositif de distribution de produit fluide comprenant un corps pourvu d'un orifice de distribution, un réservoir contenant du produit fluide et un gaz propulseur, une valve doseuse assemblée sur ledit réservoir, ledit réservoir étant déplaçable dans ledit corps pour actionner la valve doseuse et distribuer une dose de produit fluide à travers ledit orifice de distribution, ladite valve doseuse comportant une soupape coulissant dans ladite valve doseuse lors de l'actionnement, ledit dispositif comportant au moins un élément d'étanchéité pour former une étanchéité au fluide, au moins un élément d'étanchéité dudit dispositif comportant du COC élastomère.

Avantageusement, ladite valve doseuse est assemblée sur ledit réservoir avec interposition d'un joint de col.

Avantageusement, ladite valve doseuse comporte au moins un joint interne coopérant de manière étanche avec ladite soupape.

Avantageusement, ladite valve doseuse comporte un joint interne supérieur et un joint interne inférieur définissant entre eux une chambre de dosage de ladite valve doseuse.

Avantageusement, ledit joint de col et/ou ledit joint interne supérieur et/ou ledit joint interne inférieur comporte(nt) du COC élastomère.

Avantageusement, ledit au moins un élément d'étanchéité est constitué de COC élastomère.

Avantageusement, ledit produit fluide est un produit fluide pharmaceutique comportant au moins un produit actif.

Avantageusement, ledit gaz propulseur comporte des gaz HFA du type HFA 134a et/ou HFA 227.

Avantageusement, une bague est associée à la valve doseuse, au moins un élément d'étanchéité en COC élastomère étant surmoulé sur une partie de ladite valve doseuse et/ou de ladite bague.

Avantageusement, le COC élastomère est un copolymère élastomère ayant une température de transition vitreuse comprise entre - 10°C et 15 °C, une température de fusion cristalline comprise entre 50°C et 120°C, une cristallinité en poids comprise entre 5% et 40% et une teneur en norbornène comprise entre 2 et 15 mol%.

Ces avantages et d'autres de la présente invention apparaitront plus clairement au cours de la description détaillée suivante d'un mode de réalisation avantageux de celle-ci, faite en référence aux dessins joints, donnés à titre d'exemple non limitatif, et sur lesquels
La figure 1 est une vue schématique en section transversale d'un dispositif de distribution de produit fluide du type inhalateur à dose mesurée (MDI), et
La figure 2 est une vue schématique en section transversale d'un réservoir sur lequel est assemblée une valve doseuse, selon un mode de réalisation avantageux de la présente invention.

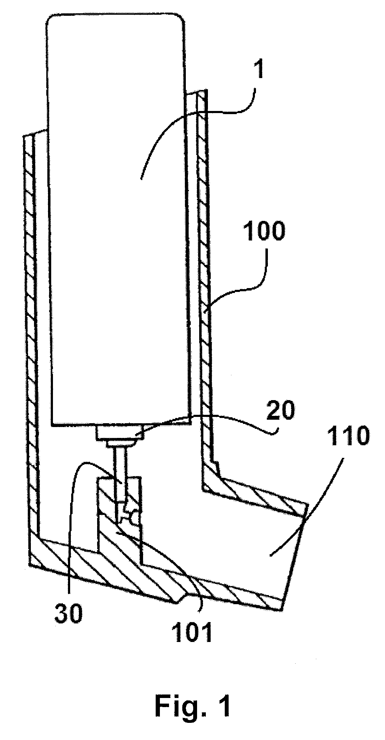

En se référant à la figure 1, il est décrit un inhalateur à dose mesurée appelé généralement pMDI, qui classiquement comporte un corps externe 100 pourvu d'un orifice de distribution 110, généralement un embout buccal. A l'intérieur de ce corps est disposé un réservoir 1 sur lequel est montée une valve doseuse 20. Une soupape 30 coulisse dans ladite valve doseuse 20 pour distribuer une dose de produit fluide à chaque actionnement. Le corps 100 comporte un puits 101 qui reçoit la soupape 30 et qui crée un passage de liaison entre la sortie de la soupape 30 et ledit orifice de distribution 110. De manière classique, pour actionner un tel dispositif, l'utilisateur appuie sur le fond du réservoir 1 pour enfoncer celui-ci axialement à l'intérieur du corps 100, ce qui provoque le coulissement étanche de la soupape 30 vers l'intérieur de la valve doseuse 20 provoquant ainsi la distribution d'une dose de produit fluide. A l'intérieur du réservoir, le produit fluide, qui contient généralement un ou plusieurs produit(s) actif(s), est associé à un gaz propulseur, de préférence un gaz du type HFA, par exemple HFA 134a et/ou HFA 227.

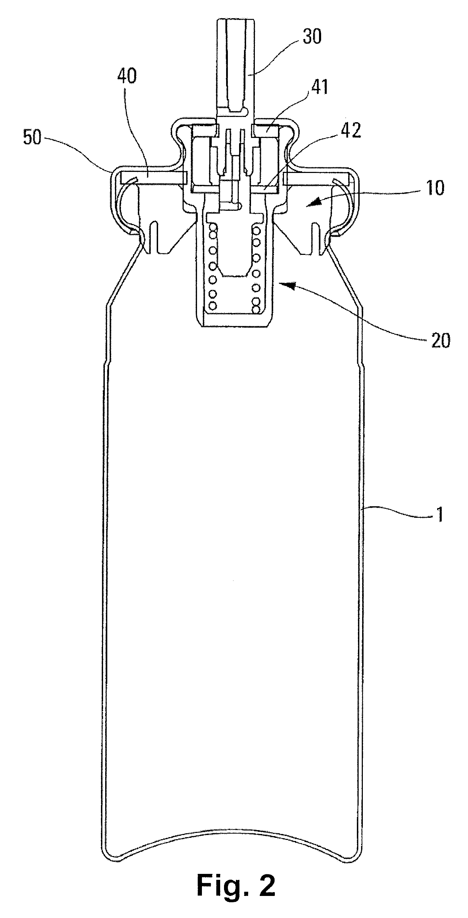

La figure 2 représente une valve doseuse selon un mode de réalisation particulier qui est avantageux. Il est entendu que la présente invention n'est pas limitée à ce type de valve doseuse, mais qu'elle s'applique à tout type de valve doseuse utilisable dans les pMDI. Une valve doseuse 20 est donc assemblée sur le réservoir 1 comme visible sur la figure 2. Cet assemblage peut être réalisé au moyen d'une bague de fixation 50, qui en l'occurrence est une bague sertie, mais qui pourrait également être une bague encliquetable ou vissable. La valve doseuse 20 comporte classiquement un corps de valve à l'intérieur duquel coulisse une soupape 30. Cette soupape 30 est sollicitée par un ressort vers sa position de repos. De manière connue, un joint d'étanchéité appelé « joint de col » 40 est interposé entre la bague de fixation 50 et le col du réservoir 1 lors de l'assemblage de la valve doseuse 20 sur le réservoir 1, pour assurer l'étanchéité au niveau du col du réservoir. De plus, la valve doseuse comporte au moins un, en l'occurrence deux, joints d'étanchéité internes 41, 42 qui coopèrent de manière étanche avec la soupape 30. Ainsi, comme représenté sur la figure 2, la valve comporte un joint interne supérieur 41 et un joint interne inférieur 42, les termes « inférieur » et « supérieur » se référant à l'orientation de la figure 2, c'est-à-dire avec la valve 20 disposée au-dessus du réservoir 1. Entre ces deux joints internes 41, 42 est définie une chambre de dosage et lorsque la soupape 30 est enfoncée dans la valve, le contenu de cette chambre de dosage est expulsé à travers la soupape, de manière classique. Une bague 10 peut être interposée entre le joint de col et le corps de valve pour limiter les contacts entre le produit actif et le joint de col 40, mais également pour limiter le volume mort dans cet endroit du dispositif. Cette bague, lorsque présente, peut être de forme et de matériau quelconque approprié.

Selon l'invention, au moins un des éléments d'étanchéité, c'est-à-dire au moins un du joint de col 40, du joint interne supérieur 41 et du joint interne inférieur 42 comporte du COC élastomère. De préférence, les trois joints ci-dessus sont réalisés avec ce matériau. Avantageusement, le COC élastomère forme le seul matériau de base, mais on pourrait envisager de réaliser un alliage de COC élastomère avec un ou plusieurs autres matériaux, notamment du type élastomère.

Le COC élastomère est notamment fabriqué et commercialisé par la société TOPAS.

Le COC est un copolymère formulé avec un cycle norbornène et du polyéthylène. Le norbornène est issu de la synthèse d'éthylène et d'un cyclo-pentadiène. Le COC classique est un matériau substantiellement rigide. Le COC élastomère est alors un COC où la part de polyéthylène est plus importante, ce qui confère des propriétés élastomériques audit matériau. Le COC élastomère n'est donc pas un mélange ou un alliage de COC classique avec un matériau élastomère, mais un matériau en tant que tel, qui présentent certaines propriétés similaires aux élastomères.

Le COC élastomère est un matériau ayant une température de transition vitreuse comprise entre - 10°C et 15 °C, une température de fusion cristalline comprise entre 50°C et 120°C, une cristallinité en poids comprise entre 5% et 40% et une teneur en norbornène comprise entre 2 et 15 mol%.

Les avantages du COC élastomère sont nombreux.

D'une part, il présente une nature chimique très neutre car, contrairement aux autres matériaux élastomères, il ne comporte pas de double liaison ouverte ou disponible susceptible de réagir.

Le COC élastomère a également un niveau d'extractibles très bas, c'est-à-dire que très peu de particules appelées extractibles sortent de joints réalisés en COC élastomère, même lorsqu'ils sont en contact avec des gaz propulseurs du type HFA, qui sont particulièrement agressifs. En particulier, le COC élastomère ne présente pas d'acides gras en tant qu'extractibles, contrairement aux thermoplastiques élastomères et aux élastomères. Les extractibles existants avec le COC élastomère comportent donc principalement des antioxydants.

Le tableau ci-dessous démontre que le niveau d'extractibles est nettement inférieur pour le COC-E par rapport au TPE (thermoplastique élastomère), qui dans cet exemple comparatif, est formé d'un mélange de 50% de butyle et de 50% de polyéthylène :

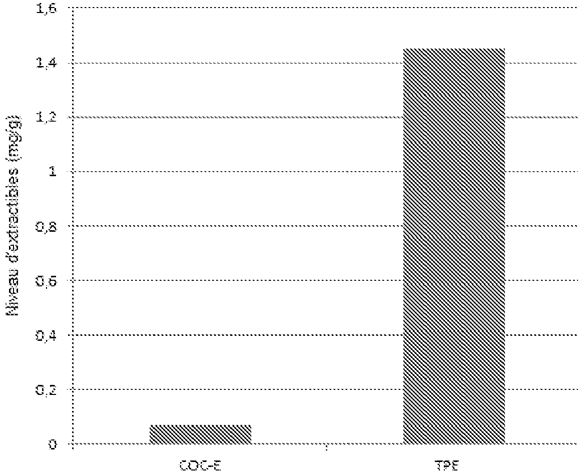

Les matériaux élastomères présentent pour leur part des niveaux d'extractibles encore nettement supérieurs, comme par exemple le nitrile qui a un niveau d'extractibles d'environ 14 mg/g ou l'EPDM qui a un niveau d'extractibles compris entre 1,4 et 5,3 mg/g.

Le COC élastomère présente également des propriétés barrière à la vapeur d'eau élevées, ainsi que des propriétés mécaniques qui sont tout à fait adaptées pour réaliser des joints de valve, notamment la dureté et le module de Young. En particulier, il offre des performances d'étanchéité (fuites statiques du propulseur, barrière à la vapeur d'eau) comparables à un matériau thermoplastique élastomère, tel que le TPE décrit ci-dessus (50% butyle et 50% polyéthylène), et des performances supérieures aux élastomères, tels que par exemple l'EPDM.

Le COC élastomère présente également une bonne résistance à l'abrasion et est susceptible de surmoulage cohésif avec d'autres polymères du type polyoléfine. En particulier, un joint en COC élastomère peut être surmoulé sur une partie de la valve et/ou une partie de la bague 10, notamment lorsque celle-ci est en matériau de même nature chimique, tel que le COC.

Le COC élastomère présente également une bonne compatibilité avec les produits actifs de type pharmaceutique, car il n'y a pas de relarguage d'ions, pas de trace métallique, il comporte des surfaces hydrophobes, de sorte qu'il y a moins d'absorption, et enfin il est de conception flexible aisée, c'est-à-dire qu'il est facile de réaliser des joints de forme quelconque à partir de ce matériau.

Par exemple et de manière non limitative, le produit COC-E X1 T6, commercialisé par la société TOPAS ADVANCED POLYMERS est un matériau adapté pour la présente invention.

De manière surprenante, il s'est avéré que ce matériau améliore le fonctionnement des valves doseuses, diminue les interactions entre le matériau et le produit actif et/ou le gaz propulseur, tout en rendant moins difficile ou compliqué et donc moins coûteux la fabrication et l'assemblage des valves et des inhalateurs dans lesquels ces valves sont utilisées.

Bien que la présente invention ait été décrite en référence à un mode de réalisation avantageux de celle-ci, il est entendu qu'elle n'est pas limitée par celui-ci mais que toutes modifications utiles peuvent y être apportées sans sortir du cadre de la présente invention tel que défini par les revendications annexées.

## Revendications

1. Dispositif de distribution de produit fluide comprenant un corps (100) pourvu d'un orifice de distribution (110), un réservoir (1) contenant du produit fluide et un gaz propulseur, une valve doseuse (20) assemblée sur ledit réservoir (1), ledit réservoir (11) étant déplaçable dans ledit corps (100) pour actionner la valve doseuse (20) et distribuer une dose de produit fluide à travers ledit orifice de distribution (110), ladite valve doseuse (20) comportant une soupape (30) coulissant dans ladite valve doseuse (20) lors de l'actionnement, ledit dispositif comportant au moins un élément d'étanchéité (40, 41, 42) pour former une étanchéité au fluide, **caractérisé en ce qu'**au moins un élément d'étanchéité (40, 41, 42) dudit dispositif comporte du COC élastomère.

2. Dispositif selon la revendication 1, dans lequel ladite valve doseuse (20) est assemblée sur ledit réservoir (1) avec interposition d'un joint de col (40).

3. Dispositif selon la revendication 1 ou 2, dans lequel ladite valve doseuse (20) comporte au moins un joint interne (41, 42) coopérant de manière étanche avec ladite soupape (30).

4. Dispositif selon la revendication 3, dans lequel ladite valve doseuse (20) comporte un joint interne supérieur (41) et un joint interne inférieur (42) définissant entre eux une chambre de dosage de ladite valve doseuse (20).

5. Dispositif selon la revendication 2 et selon l'une des revendications 3 ou 4, dans lequel ledit joint de col (40) et/ou ledit joint interne supérieur (41) et/ou ledit joint interne inférieur (42) comporte(nt) du COC élastomère.

6. Dispositif selon l'une quelconque des revendications précédentes, dans lequel ledit au moins un élément d'étanchéité (40, 41, 42) est constitué de COC élastomère.

7. Dispositif selon l'une quelconque des revendications précédentes, dans lequel ledit produit fluide est un produit fluide pharmaceutique comportant au moins un produit actif.

8. Dispositif selon l'une quelconque des revendications précédentes, dans lequel ledit gaz propulseur comporte des gaz HFA du type HFA 134a et/ou HFA 227.

9. Dispositif selon l'une quelconque des revendications précédentes, dans lequel une bague (10) est associée à la valve doseuse (20), au moins un élément d'étanchéité (40, 41, 42) en COC élastomère étant surmoulé sur une partie de ladite valve doseuse et/ou de ladite bague.

10. Dispositif selon l'une quelconque des revendications précédentes, dans lequel le COC élastomère est un copolymère élastomère ayant une température de transition vitreuse comprise entre - 10°C et 15 °C, une température de fusion cristalline comprise entre 50°C et 120°C, une cristallinité en poids comprise entre 5% et 40% et une teneur en norbornène comprise entre 2 et 15 mol%.

## Patentansprüche

1. Ausgabevorrichtung für ein fluides Produkt, aufweisend einen Körper (100), der mit einer Ausgabeöffnung (110) versehen ist, einen Behälter (1), der fluides Produkt und ein Treibgas enthält, ein Dosierventil (20), das auf dem Behälter (1) montiert ist, wobei der Behälter (11) in dem Körper (100) verschiebbar ist, um das Dosierventil (20) zu betätigen und durch die Ausgabeöffnung (110) eine Dosis von fluidem Produkt auszugeben, wobei das Dosierventil (20) ein federgespanntes Ventil (30) aufweist, welches bei Betätigung in dem Dosierventil (20) gleitet, wobei die Vorrichtung mindestens ein Dichtigkeitselement (40, 41, 42) zum Bilden einer Dichtigkeit gegenüber dem Fluid aufweist, **dadurch gekennzeichnet, dass** mindestens ein Dichtigkeitselement (40, 41, 42) der Vorrichtung COC-Elastomer umfasst.

2. Vorrichtung nach Anspruch 1, wobei das Dosierventil (20) unter Einschiebung einer Kragendichtung (40) auf dem Behälter (1) montiert ist.

3. Vorrichtung nach Anspruch 1 oder 2, wobei das Dosierventil (20) mindestens eine interne Dichtung (41, 42) aufweist, die dichtend mit dem federgespannten Ventil (30) zusammenwirkt.

4. Vorrichtung nach Anspruch 3, wobei das Dosierventil (20) eine obere interne Dichtung (41) und eine untere interne Dichtung (42) aufweist, die zwischen sich eine Dosierkammer des Dosierventils (20) definieren.

5. Vorrichtung nach Anspruch 2 und nach einem der Ansprüche 3 oder 4, wobei die Kragendichtung (40) und/oder die obere interne Dichtung (41) und/oder die untere interne Dichtung (42) COC-Elastomer aufweist (aufweisen).

6. Vorrichtung nach einem der vorhergehenden Ansprüche, wobei das mindestens eine Dichtigkeitselement (40, 41, 42) aus COC-Elastomer gebildet ist.

7. Vorrichtung nach einem der vorhergehenden Ansprüche, wobei das fluide Produkt ein pharmazeutisches fluides Produkt ist, welches mindestens ein Wirkprodukt aufweist.

8. Vorrichtung nach einem der vorhergehenden Ansprüche, wobei das Treibgas HFA-Gas vom Typ HFA 134a und/oder HFA 227 umfasst.

9. Vorrichtung nach einem der vorhergehenden Ansprüche, wobei ein Ring (10) mit dem Dosierventil (20) verbunden ist, wobei mindestens ein Dichtigkeitselement (40, 41, 42) aus COC-Elastomer auf einem Teil des Dosierventils und/oder des Rings aufgegossen ist.

10. Vorrichtung nach einem der vorhergehenden Ansprüche, wobei das COC-Elastomer ein elastomeres Copolymer mit einer Glasübergangstemperatur zwischen -10 °C und 15 °C, einem kristallinen Schmelzpunkt zwischen 50 °C und 120 °C, einem Gewichtskristallinitätsgrad zwischen 5 % und 40 % und einem Norbornengehalt zwischen 2 und 15 Mol-% ist.

## Claims

1. A fluid dispenser device comprising: a body (100) that is provided with a dispenser orifice (110); a reservoir (1) containing fluid and a propellant gas; and a metering valve (20) that is assembled on said reservoir (1); said reservoir (1) being movable in said body (100) so as to actuate the metering valve (20) and dispense a dose of fluid through said dispenser orifice (110), said metering valve (20) including a valve member (30) that slides in said metering valve (20) during actuation; said device further comprising at least one sealing element (40, 41, 42) so as to form a leaktight seal, the device being **characterized in that** at least one sealing element (40, 41, 42) of said device comprises COC elastomer.

2. A device according to claim 1, wherein said metering valve (20) is assembled on said reservoir (1) with a neck gasket (40) interposed therebetween.

3. A device according to claim 1 or claim 2, wherein said metering valve (20) includes at least one internal gasket (41, 42) that co-operates in leaktight manner with said valve member (30).

4. A device according to claim 3, wherein said metering valve (20) includes an upper internal gasket (41) and a lower internal gasket (42), defining between them a metering chamber of said metering valve (20).

5. A device according to claim 2 and according to claim 3 or claim 4, wherein said neck gasket (40) and/or said upper internal gasket (41) and/or said lower internal gasket (42) comprise(s) COC elastomer.

6. A device according to any preceding claim, wherein said at least one sealing element (40, 41, 42) is constituted by COC elastomer.

7. A device according to any preceding claim, wherein said fluid is a pharmaceutical fluid containing at least one active substance.

8. A device according to any preceding claim, wherein said propellant gas comprises HFA gases of the HFA 134a and/or HFA 227 type.

9. A device according to any preceding claim, wherein a ring (10) is associated with the metering valve (20), at least one sealing element (40, 41, 42) made of COC elastomer being over-molded on a portion of said metering valve and/or of said ring.

10. A device according to any preceding claim, wherein COC elastomer is a copolymer elastomer having a glass transition temperature lying in the range -10°C to 15°C, a crystalline melting point lying in the range 50°C to 120°C, a crystallinity by weight lying in the range 5% to 40%, and a norbornene content lying in the range 2 mol% to 15 mol%.
